# EUROPEAN PATENT SPECIFICATION

(11) **EP 2 180 883 B1**
(45) Date of publication and mention of the grant of the patent: **11.01.2017**
(21) Application number: 07786262.1
(22) Date of filing: 23.07.2007
(51) Int. Cl.: A61K 9/20, A61K 31/4422

(54) **PHARMACEUTICAL COMPOSITION CONTAINING DIHYDROPYRIDINE CALCIUM CHANNEL ANTAGONIST AND METHOD FOR THE PREPARATION THEREOF**
PHARMAZEUTISCHE ZUSAMMENSETZUNG MIT EINEM DIHYDROPYRIDIN-CALCIUMKANAL-ANTAGONIST UND VERFAHREN ZU SEINER HERSTELLUNG
COMPOSITION PHARMACEUTIQUE CONTENANT UN ANTAGONISTE DES CANAUX CALCIQUES DE TYPE DIHYDROPYRIDINE ET SON PROCÉDÉ DE PRÉPARATION

(43) Date of publication of application: 05.05.2010
(73) Proprietor: Pharmathen S.A., Pallini Attikis 15351 (GR)
(72) Inventor: KARAVAS, Evangelos, GR-153 51 Pallini Attikis (GR); KOUTRIS, Efthimios, GR-153 51 Pallini Attikis (GR); KOUTRI, Ioanna, GR-153 51 Pallini Attikis (GR); STATHAKI, Eleni, Gr-153 51 Attikis (GR)
(86) International application number: PCT/EP2007/006517
(87) International publication number: WO 2009/012791

(56) References cited:
- EP-A- 0 487 335
- EP-A- 1 013 271
- WO-A-2005/053689
- WO-A2-2008/068777
- KERC J ET AL: "USE OF HYDROPHILIC CARRIERS IN ENHANCEMENT OF FELODIPINE DISSOLUTION" ACTA PHARMACEUTICA JUGOSLAVICA, vol. 41, no. 3, 1991, pages 259-266, XP008089880 ISSN: 0001-6667

## Description

### TECHNICAL FIELD OF THE INVENTION

The present invention relates to improved dosage forms such as tablets and capsules and in particular to a formulation for oral administration with enhanced bioavailability comprising a therapeutically effective quantity of a dihydropyridine calcium channel antagonist, and more particularly Lercanidipine or salt, derivative and polymorph thereof and a method for the preparation thereof.

### BACKGROUND OF THE INVENTION

Dihydropyridine calcium channel antagonist compounds, such as amlopidipine, nifedipine, lacidipine and lercanidipine, are compounds known to be extremely useful among others in the treatment of hypertension and coronary disease.

Lercanidipine (methyl 1, 1-N-trimethyl-N-(3, 3-diphenylpropyl) amino ethyl 1, 4-dihydro-6-dimethyl (3-nitrophenyl) pyridine-3, 5-dicarboxylate) is a highly lipophilic dihydropyridine calcium antagonist with long duration of action and high vascular selectivity. It is normally used in a dose of 10mg to 20mg once daily, the maximum dose being about 30mg daily. Lercanidipine is rapidly absorbed following oral administration and peak plasma levels occurring 1, 5-3 hours following dosing, but it undergoes extensive first pass metabolism.

Dihydropyridine calcium channel antagonists have low water solubility and that result to low bioavailability of the active ingredient.

Drugs with low solubility in water (by which is meant having a solubility of less than 0.1 percent by weight in water at 20 °C.) cause additional formulation problems due to their poor rate and extent of dissolution in aqueous media, including gastrointestinal fluids, which results in low absorption into systemic circulation after oral ingestion.

In order to make a composition containing such a drug that will enable maximum absorption from the gastrointestinal tract, it is necessary to incorporate in the composition a feature that increases the solubility of the drug to enable it to dissolve in the gastrointestinal fluids.

Various methods are already known for the industrial preparation of oral dosage forms comprising a dihydropyridine calcium channel antagonist, and in particular Lercanidipine or a pharmaceutical acceptable salt thereof as an active ingredient due to its useful therapeutical properties. However, the prior art has encountered substantial difficulties in the production of the oral solid formulations of a desirable bioavailability due to the poor solubility of said active ingredient.

It is known that active compounds in amorphous form often have a higher bioavailability than corresponding crystalline active compounds. DE-A-3 024 858 discloses a dosage form comprising nicardipine, a sparingly soluble dihydropyridine, used in its amorphous form in order to increase dissolution and absorption. Amorphous active ingredients usually should be carefully formulated because they have a tendency to recrystallize, resulting to bioavailability that is not reproducible or decreases significantly after certain storage periods due to degradation products.

EP 0 385 582 discloses nifedipine composition having a particle size of less than 100 microns. Although the control of the dissolution of nifedipine is achieved by processing the material to a large specific surface area, the small crystals of the active ingredient have the tendency to agglomerate and reform to larger particle sizes.

EP 0 557 244 discloses compositions which contain nifedipine which has been micronized to small crystals to increase solubility, along with a hydrophilic gel-forming polymer to slow-down and control the rate of dissolution and absorption. However, the smallest size to which nifedipine can be micronized using conventional equipment is about 1 micron, and this particle size is still not small enough to enable full dissolution and absorption of the nifedipine.

Moreover, unless the crystal size is carefully controlled to be the same in every batch of tablets, release characteristics may vary from batch to batch.

GB 1 456 618 discloses improving the dissolution and absorption of nifedipine by preparation of a solid solution of nifedipine in polyethylene glycol in the presence of a surfactant agent.

EP 0 448 091 discloses a dihydropyridine with a surfactant but large quantities of surfactants usually cause irritation to the stomach of the patients.

In addition, the use of surfactants, solubilizing agents and certain excipients which have a particular surface frequently leads to administration forms in which the products are undesirably large. To facilitate swallowing, such tablets or capsules are frequently converted into specific forms, such as, for example, ellipsoids or longitudinal shapes, but this also no longer gives satisfactory results in products weighing more than 400 mg. More frequent taking of smaller products is also not a satisfactory solution.

WO 2005/053689 A discloses a controlled release composition comprising lercanidipine dissolved or dispersed in a solid vehicle forming a solid dispersion.

Although each of the above patents, represents an attempt to overcome the instability problems associated with pharmaceuticals compositions comprising a dihydropyridine calcium channel antagonist, there still exists a need for improving the bioavailability of such pharmaceutical compositions.

### SUMMARY OF THE INVENTION

It is, therefore, an object of the present invention to provide an improved solid dosage formulation for oral administration containing a dihydropyridine calcium channel antagonist, and in particular Lercanidipine or salt thereof as an active ingredient, which overcomes the deficiencies of the prior art and enhances the bioavailability of the active ingredient.

Another aspect of the present invention is to provide a solid dosage formulation for oral administration containing a dihydropyridine calcium channel antagonist, and in particular Lercanidipine or salt thereof as an active ingredient, which is bioavailable and effective with sufficient self-life, good pharmacotechnical properties enhancing patient compliance and reducing possible side effects.

Moreover, another aspect of the present invention is to provide a solid dosage formulation for oral administration containing a dihydropyridine calcium channel antagonist, and in particular Lercanidipine or salt thereof as an active ingredient, which can be prepared in dosage forms of different strength by proportionally adjusting the quantities of the excipients and the active ingredient, thereby providing a pharmacotechnical linearity, without affecting the dissolution profile and bioavailability of the active ingredient.

A further aspect of the present invention is to provide a method for the preparation of a stable solid dosage formulation for oral administration containing a dihydropyridine calcium channel antagonist, and in particular Lercanidipine or salt thereof as an active ingredient, thereby enhancing the bioavailability of the active ingredient, being stable over a long period of time and improving the pharmacotechnical characteristics of the composition.

In accordance with the above objects of the present invention. there is provided a process for the preparation of solid dosage forms for oral administration such as tablets, capsules and sachets, containing dihydropyridine calcium channel antagonist Lercanidipine or a pharmaceutical acceptable salt, derivative and polymorph thereof as an active ingredient and an amount from 5% to 25% by weight of colloidal silicon dioxide, which comprises:
- dissolving the total quantity of said active ingredient, a portion of a total quantity of colloidal silicon dioxide to enhance bioavailability and a binder into a water/EtOH solvent;
- adding to the formed solution the remaining portion of colloidal silicon dioxide and an optional excipient such as a diluent, a binder, a disintegrant, a glidant, a lubricant and wet granulating;
- dissolving a wetting agent into a small quantity of water/EtOH solvent and kneading with the first solution;
- drying the wetted mass;
- sieving the dried mass and adding to the sieved mixture the total quantities of at least one optional excipient such as a binder, a wetting agent, a diluent, a disintegrant, a lubricant and/or a glidant and mixing until uniform, and
- formulating the resulting mixture in a solid dosage form either by compressing it into a desired tablet form or by filling capsules or sachets.

Also provided is a pharmaceutical composition for oral administration obtainable by said process. Further preferred embodiments of the present invention are defined in dependent claims 3-12.

Other objects and advantages of the present invention will become apparent to those skilled in the art in view of the following detailed description.

### BRIEF DESCRIPTION OF THE DRAWINGS

Figs. 1 and 2 show average plasma values for the composition of Example 1 according to the present invention.
Figs. 3 and 4 show average plasma values for the composition of Example 3 according to the present invention.
Figs. 5, 6, and 7 show X-RD spectrums of amorphous Lercanidipine HCl, placebo of composition of Example 3, composition of Example 3 according to the present invention.
Fig. 8 shows SEM of amorphous Lercanidipine HCl.
Fig. 9 shows SEM of fine dispersion of amorphous Lercanidipine HCl and colloidal silicon dioxide according to the present invention.

### DETAILED DESCRIPTION OF THE INVENTION

For the purposes of the present invention, a pharmaceutical composition comprising a poorly soluble active ingredient (Dihydropyridine calcium channel antagonist Lercanidipine or pharmaceutical acceptable salt thereof) is considered to be "stable" if said ingredient degradates less or more slowly than it does on its own and/or in known pharmaceutical compositions.

The active ingredient ( Lercanidipine or a pharmaceutical acceptable salt thereof) contained in a dosage form is "enhanced bioavailable", if when administered in a dosage form is released from the dosage form, absorbed and subsequently reaches higher concentration levels in plasma than the marketed products containing the same quantity of the same active ingredient and intended for the same use.

Although the pharmaceutical composition may be in various forms, the preferred solid forms are tablets, capsules and caplets.

It has been surprisingly found that the object of the present invention is achieved by employing colloidal silicon dioxide such as Aerosil™ in order to enhance the bioavailability of the active ingredient.

As already mentioned dihydropyridine calcium channel antagonists such as Lercanidipine HCl have very poor solubility thus reflecting in poor bioavailability of the active substance.

Colloidal silicon dioxide is a submicroscopic fumed silica with a particle size of about 15nm. It is a light, loose, bluish-white-colored, odourless, tasteless, nongritty amorphous powder. Colloidal silicon dioxide is widely used in pharmaceuticals. Its small particle size and large specific surface area give it desirable flow characteristics that are exploited to improve the flow properties of dry powders

When colloidal silicon dioxide is incorporated in a pharmaceutical composition according to the present invention a fine dispersion of amorphous particles of the active ingredient on the surface of colloidal silicon dioxide is being formed resulting in a one-phase system. Said one-phase system improves the solubility of the active ingredient.

The active ingredient (dihydropyridine calcium channel antagonists such as Lercanidipine or salt, derivative and polymorph thereof) and a suitable amount of colloidal silicon dioxide such as Aerosil™ are being dissolved in a solvent in order to form a fine dispersion, and subsequently a binder is being admixed. The remaining portion of the colloidal silicon dioxide and an optional excipient is being added in the solution and wet granulating. After drying the wetted mass and sieving the dried mass, any optional additional excipient is then added. The composition is then mixed until uniform. The resulting composition may then be compressed.

Moreover, any excipient may optionally be added to the above composition, provided that they are compatible with the active ingredient of the composition, in order to overcome problems associated with the poor flow properties and unfavorable pharmacotechnical characteristics of these substances, and in order to increase the stability of the drug and the self-life of the pharmaceutical product, and provide a product exhibiting excellent bioavailability.

The present invention can be applied in the formulation of tablets, capsules, caplets, sachets or other solid dosage forms for oral or sub-lingual administration of lercanidipine, having solubility and bioavailability problems.

Furthermore, it is possible to prepare dosage forms of different strength using appropriate quantity of the same composition, thereby limiting the cost of production and minimizing the number, and consequently the cost, of clinical studies required for the approval of the product by the authorities.

The manufacturing process for preparation according to the present invention is simpler and inexpensive in comparison to any other conventional method.

In an embodiment, the present invention provides a pharmaceutical composition comprising from about 0.5% to 30% by weight of Lercanidipine or salt thereof. The weight ratio of Lercanidipine or salt thereof to Colloidal silicon dioxide is preferably 10:1 to 1:60.

More preferred pharmaceutical compositions according to the present invention comprise 7% to 20% by weight of Colloidal silicon dioxide, such as Aerosil™.

A binder. may be generally present in an amount of up to about 15 % by weight and the wetting agent, may generally be present in an amount of up to about 5 % by weight.

The preferred pharmaceutical compositions are in the form of solid dosage forms for oral or sub-lingual administration such as tablets, capsules, caplets, troches, pastilles, pills, lozenges and the like, in all shapes and sizes, coated or uncoated.

All percentages stated herein are weight percentages based on total composition weight, unless otherwise stated.

Another embodiment of the present invention is the use of the wet granulation process for the preparation of solid dosage forms for oral administration such as tablets, capsules and sachets containing Lercanidipine or salt, derivative and polymorph thereof. Said wet granulation process is as described above.

The pharmaceutical compositions of the present invention may also contain one or more additional formulation ingredients selected from a wide variety of excipients. According to the desired properties of the composition, any number of ingredients may be selected, alone or in combination, based upon their known uses in preparation of solid dosage form compositions.

Such ingredients include, but are not limited to, diluents, binders, compression aids, disintegrants, surfactants, wetting agents, antioxidants, glidants, lubricants, flavors, water scavengers, colorants, sweetener, coating agents and preservatives.

The optional excipients must be compatible with the dihydropyridine calcium channel antagonist or the salt thereof so that it does not interfere with it in the composition.

Diluents may be, for example, calcium carbonate, calcium phosphate dibasic, calcium phosphate tribasic, calcium sulfate, microcrystalline cellulose, microcrystalline silicified cellulose, powdered cellulose, dextrates, dextrose, fructose, lactitol, lactose anhydrous, lactose monohydrate, lactose dihydrate, lactose trihydrate, mannitol sorbitol, starch, pregelatinized starch, sucrose, talc, xylitol, maltose maltodextrin, maltitol.

Binders may be, for example, acacia mucilage, alginic acid, carbomer, carboxymethylcellulose calcium, carboxymethylcellulose sodium, microcrystalline cellulose, powdered cellulose, ethyl cellulose, gelatin, liquid glucose, guar gum, hydroxyethyl cellulose, hydroxypropyl cellulose, hydroxypropylmethyl cellulose, maltodextrin, methylcellulose, polydextrose, polyethylene oxide, povidone, sodium alginate, starch paste, pregelatinized starch, sucrose.

Disintegrants may be, for example, alginic acid, carbon dioxide, carboxymethylcellulose calcium, carboxymethylcellulose sodium, microcrystalline cellulose, powdered cellulose, croscarmelose sodium, crospovidone, sodium docusate, guar gum, hydroxypropyl cellulose, methylcellulose, polacrilin potassium, poloxamer, povidone, sodium alginate, sodium glycine carbonate, sodium laulyl sulfate, sodium starch glycolate, starch, pregelatinized starch.

Wetting agents may be, polyoxyethylene-polyoxypropylene co-polymers and block co-polymers, commercially available as Pluronic™ or Poloxamer™, ethoxylated cholesterins, commercially available as Solulan™ vitamin derivatives, e. g. vitamin E derivatives such as tocopherol polyethylene glycol succinate (TPGS), sodium dodecylsulfate or sodium laurylsulfate; a bile acid or salt thereof, for example cholic acid, glycolic acid or a salt.

Glidants may be, for example, calcium silicate, powdered cellulose, starch, talc, colloidal silicon dioxide.

Lubricants may be e.g. polyethylene glycol 4000, polyethylene glycol 6000, sodium lauryl sulfate, starch, talc.

The following examples illustrate preferred embodiments in accordance with the present invention without limiting the scope of the invention:

### EXAMPLES

(Examples 1 and 2 are not according to the invention)

**Example 1: Tablet of 20 mg Lercanidipine (Comp. 1)**

| **Ingredients** | **%** | **20mg Tablet** |
|---|---|---|
| **Internal Phase** | | |
| Lercanidipine HCl | 10.00 | 20.00 |
| Microcellac | 40.00 | 80.00 |
| Microcrystalline Cellulose | 25.00 | 50.00 |
| Starch 1500 | 17.00 | 34.00 |
| Primojel | 2.00 | 4.00 |
| HPC | 5.00 | 10.00 |
| *Purified Water* | | *30. 00* |
| *EtOH* | | *24. 00* |

| **External phase** | | |
|---|---|---|
| Mg Stearate | 1.00 | 2.00 |
| | **Total weight** | 200.00 |

Tablets of the above formulation were prepared according to the following manufacturing process: HPC was dissolved in a water/EtOH solvent. Lercanidipine HCl was mixed with Microcellac, Microcrystalline Cellulose, Starch 1500 and Primojel to form a homogenous mixture. The above mixture was kneaded with the solution of HPC. The granular mass was dried. Finally Mg Stearate was added to the dried granule and mixed until complete homogeneity. The resulting granule was compressed into tablets.

The produced tablets were tested for hardness, friability, disintegration, and water content. All tests were performed according to European Pharmacopoeia 5.1 and were well within the specifications.

### Example 2 : Tablet of 20 mg Lercanidipine (Comp. 2)

| **Ingredients** | **%** | **20mg Tablet** |
|---|---|---|
| **Internal Phase** | | |
| Lercanidipine HCl | 10.00 | 20.00 |
| Lactose Monohydrate | 35.00 | 70.00 |
| Microcrystalline Cellulose | 22.50 | 45.00 |
| Starch 1500 | 15.00 | 30.00 |
| Primojel | 8.00 | 16.00 |
| Tween 20 | 1.00 | 2.00 |
| *Purified Water* | | *45.00* |
| *EtOH* | | *19.80* |

| **External phase** | | |
|---|---|---|
| Microcrystalline Cellulose | 7.50 | 15.00 |
| Mg Stearate | 1.00 | 2.00 |
| | **Total weight** | 200.00 |

Tablets of the above formulation were prepared according to the following manufacturing process: Tween 20 was dissolved in 20mg of water (solution 1). Lactose monohydrate, Lercanidipine HCl and half of Primojel's quantity were dissolved in the remaining quantity of water and EtOH and mixed (solution 2). Solution 1 and 2 were combined and subsequently a mixture of microcrystalline cellulose, Starch 1500 and the remaining quantity of Primojel were added and mixed. The granular mass was dried. Microcrystalline cellulose was added to the dried granule and mixed. Finally, Mg Stearate was added to the granule and mixed until complete homogeneity. The resulting granule was compressed into tablets.

### Example 3: Tablet of 20 mg Lercanidipine (Comp. 3)

| **Ingredients** | **%** | **20mg Tablet** |
|---|---|---|
| **Internal Phase** | | |
| Lercanidipine HCl | 10.00 | 20.00 |
| Lactose Monohydrate | 48.00 | 96.00 |
| Microcrystalline Cellulose | 8.00 | 16.00 |
| Primojel | 10.00 | 20.00 |
| PVP | 5.00 | 10.00 |
| Aerosil | 15.50 | 31.00 |
| Poloxamer | 2.50 | 5.00 |
| *Purified Water* | | *43.30* |
| *EtOH* | | *34.70* |

| **External phase** | | |
|---|---|---|
| Mg Stearate | 1.00 | 2.00 |
| | **Total weight** | 200.00 |

Tablets of the above formulation were prepared according to the following manufacturing process: Lercanidipine HCl and half of the quantity of Aerosil™ were dissolved in a water/EtOH solvent, and subsequently PVP was added. Lactose monohydrate and the remaining portion of the quantity of Aerosil™ were admixed, added to the previous solution and kneaded. Then Primojel was added to the above solution. Poloxamer was dissolved in a small quantity of water/EtOH solvent and kneaded with the previous solution. The granular mass was dried and sieved. Finally, Mg Stearate was added to the dried granule and mixed to complete homogeneity. The resulting granule was compressed into tablets.

One of the most critical pharmacotechnical tests is the dissolution test as it is strongly correlated with the bioavailability of the product. For the dissolution method a Paddles Apparatus II was run at 75rpm, 37C ± 0.5 °C, for 30min, while as dissolution medium a buffer pH=1,2 was used. Dissolution rate results for each composition tested are given in Table 1. The results show that all three compositions are not completely dissolved in about 30 minutes.

**TABLE 1: Dissolution profiles of the compositions of Examples 1, 2 and 3**

| **Time (min)** | **Composition 1** | **Composition 2** | **Composition 3** |
|---|---|---|---|
| **5** | 12,25 | 49,31 | 19,96 |
| **10** | 25,99 | 60,18 | 31,33 |
| **15** | 35,94 | 65,80 | 69,07 |
| **20** | 42,08 | 70,11 | 80,69 |
| **25** | 47,91 | 73,29 | 88,01 |
| **30** | 55,29 | 74,83 | 90,73 |

It is a generally known problem for pharmaceutical compositions of low solubility active ingredients that even though dissolution test provides satisfactory results, many times in vivo results depart from what it is expected. For this kind of drugs with low absorption (lower than 10 %) due to low solubility and high first pass metabolism the dissolution test is not so discriminative, thus only the pharmacokinetic study results are representative regarding the formulations.

Another objects of the present invention was to prepare a pharmaceutical composition that is stable, said active ingredient does not degradates and remains in amorphous form for a long period of storage time. Therefore, prior to the clinical trials, the three compositions were packed in PVC/PE/PVDC Aluminum blisters and exposed to normal (25°C±2°C/60%±5% RH), and accelerated (40°C±2°C/75%±5% RH) stability studies according to the current ICH guidelines. The stability results after six months are shown in the table below.

The results show that Lercanidipine is more stable when colloidal silicon dioxide is incorporated in the formulation.

The specific tests and results are described in the stability table (TABLE 2).

**TABLE 2: Stability results for compositions 1, 2 and 3 directly after preparation and after 6 months of storage in normal and accelerated conditions.**

| **0 MONTHS** | | | |
|---|---|---|---|
| **IMPURITIES** | **Comp 1** | **Comp 2** | **Comp 3** |
| Imp A NMT 0.15% | ND | ND | ND |
| Imp B NMT 0.15% | ND | ND | ND |
| Imp C NMT 0.15% | 0,04% | 0,05% | 0,03% |
| Imp D NMT 0.50% | 0,04% | 0,04% | 0,04% |
| Unknown NMT 0.20% | ND | ND | ND |
| Total NMT 1.2% | 0,08% | 0,09% | 0,07% |
| | | | |

| **6 MONTHS** | | | |
|---|---|---|---|
| **IMPURITIES** | **Comp 1** | **Comp 2** | **Comp 3** |
| **25°C±2°C/60%±5% RH** | | | |
| Imp A NMT 0.15 % | ND | ND | ND |
| Imp B NMT 0.15% | ND | ND | ND |
| Imp C NMT 0.15% | 0,04% | 0,06% | 0,03% |
| Imp D NMT 0.50% | 0,04% | 0,04% | 0,04% |
| Unknown NMT 0.20% | ND | ND | ND |
| Total NMT 1.2% | 0,08% | 0,10% | 0,07% |
| | | | |

| **40°C±2°C/75%±5% RH** | | | |
|---|---|---|---|
| Imp A NMT 0.15% | ND | ND | ND |
| Imp B NMT 0.15% | ND | 0,01% | ND |
| Imp C NMT 0.15% | 0,06% | 0,06% | 0,05% |
| Imp D NMT 0.50% | 0,18% | 0,17% | 0,16% |
| Unknown NMT 0.20% | ND | ND | ND |
| Total NMT 1.2% | 0,24% | 0,24% | 0,21% |

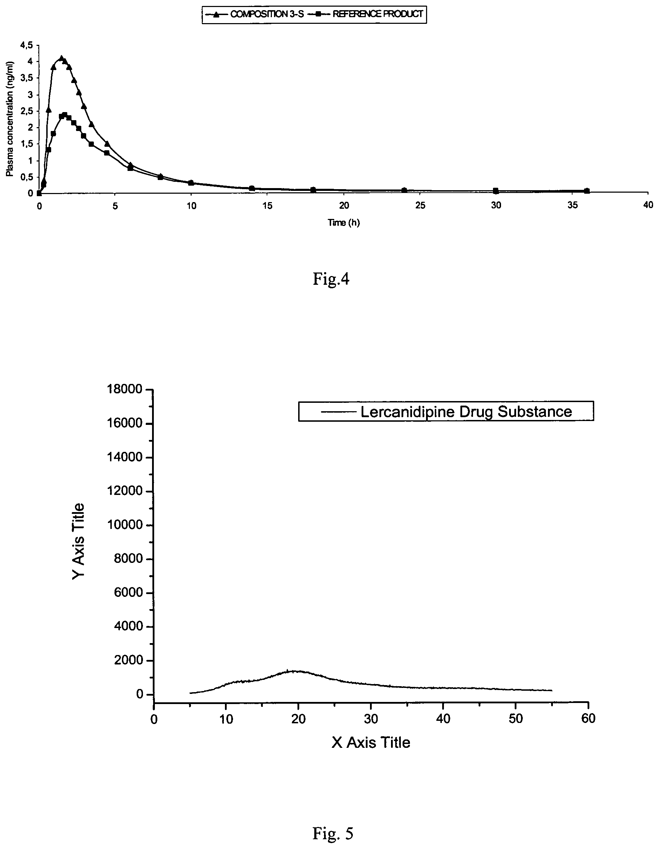

According to another aspect of the present invention, the active substance should remain in amorphous state after compression and should not convert in crystalline form.
As shown in Fig. 5 by the X-RD analysis Lercanidipine is completely amorphous since only a broad peak is recorded with a maximum at around 2θ = 20 deg. The crystal properties remain also unchanged after six months in the same conditions when the mixture is incorporated in a pharmaceutical composition with other excipients. No peaks corresponding to any crystalline form of Lercanidipine are observed directly after preparation or after 6 months storage indicating that the mixture is stabilized.

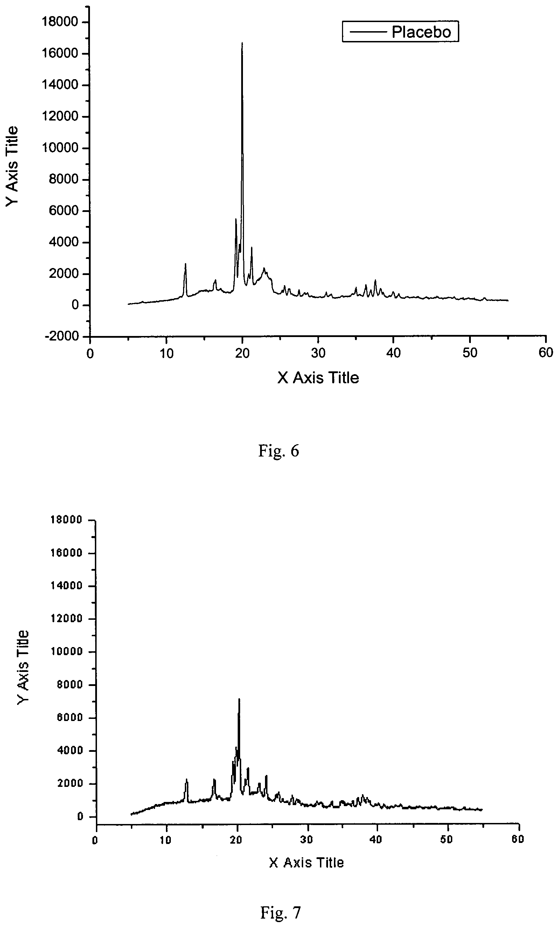

Tablets of the composition of Example 3 have the main peaks obtained at approximately 2θ=12.7, 16.6, 19.2, 19.7, 20.2, 21.4, 23.0, 36.4, 37.8 degrees which are also found in the placebo tablets (Fig. 6 and 7).

The bioavailability and pharmacokinetic profile of all three compositions of the present invention were determined in "in vivo" single-dose studies.

A single-dose study was conducted in 12 healthy volunteers using a formulation prepared with amorphous Lercanidipine HCL according to Examples 1, 2 and 3.

The reference compound was a 20mg Lercanidipine HCL tablet (Carmen 20 mg) that consists of the active ingredient, lactose, microcrystalline cellulose, sodium starch glycolate, povidone, magnesium stearate and opadry pink (Composition B).

Each patient received a single oral 20mg dose of composition 1 of Example land a tablet of Composition B equal to 20 mg of active ingredient, at different times. Blood samples were taken at different times and the plasma concentrations of Lercanidipine were determined.
In the pharmacokinetic analysis of composition 1 according to Example 1, R-Lercanidipine and S-Lercanidipine are measured separately (chiral method). Table 3 shows the main pharmacokinetic parameters obtained from the test.

**TABLE 3: Pharmacokinetic analysis of composition 1 versus reference product (B)**

| | InAUC0-t (ng h/ml) | lnAUC0-inf (ng h/ml) | Cmax (ng/ml) |
|---|---|---|---|
| R-Lercanidipine | | | |
| Ratio of least square means (comp1/B) | 50.1 | 51.5 | 57.1 |
| 90% C.I. (comp1/B) | 40.2 to 62.4 | 41.5 to 63.9 | 41.7 to 78.2 |
| Intra subject CV% | 30.4 | 29.8 | 44.4 |

| S-Lercanidipine | | | |
|---|---|---|---|
| Ratio of least square means (comp1/B) | 48.1 | 48.9 | 56.5 |
| 90% C.I. (comp1/B) | 37.4 to 61.8 | 38.1 to 62.7 | 39.3 to 81.1 |
| Intra subject CV% | 35.0 | 34.6 | 52.1 |

wherein:
C max = (peak concentration) is the highest concentration reached by the drug in plasma after dosing;
AUC₀₋ₜ =(area under the curve) is the total area under the time - plasma concentration curve, from time 0 to the last measurable concentration, as calculated by the linear trapezoidal method; it represents a measure of the bioavailability of the drug.

AUC_{0-inf} =(area under the curve) is the total area under the time- plasma concentration curve from time 0 to infinity. AUCinf is calculated as the sum of AUC 0-t plus the ratio of the last measurable plasma concentration to the elimination rate constant.
These data show that the properties of the two formulations are comparable with respect to the main pharmacokinetic parameters.

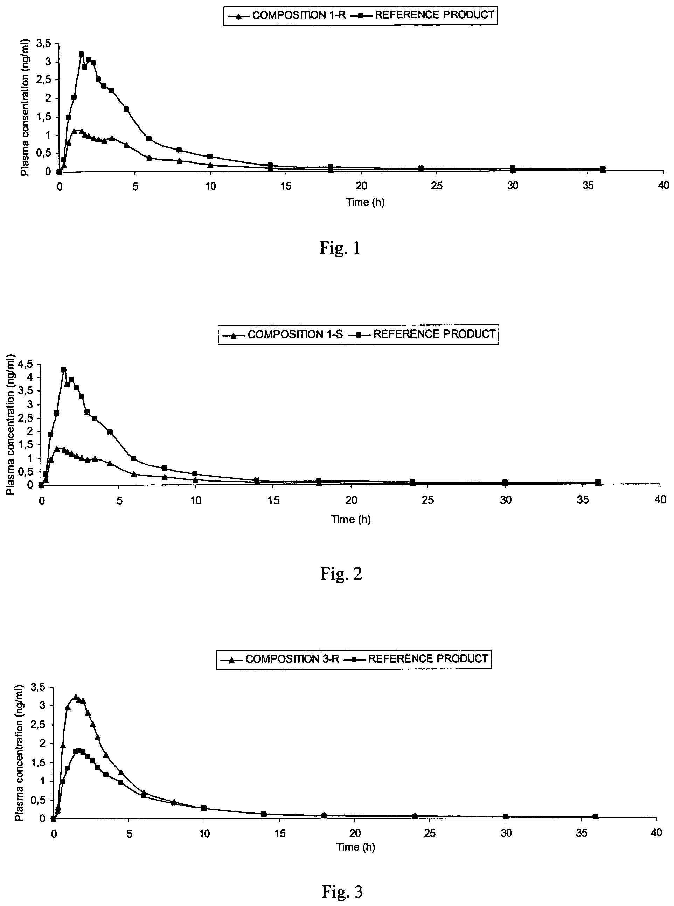

Consequently, it has been found that composition 1 (20 mg tablet) has a relative bioavailability of approximately 50% compared to the marketed Lercanidipine HCl 20 mg tablet (Fig. 1 and 2).

Moreover, a single-dose study was conducted in 12 human volunteers in a randomised two-way crossover study, comparing the dosage form of composition of Example 2 with the dosage form of composition B. Plasma samples were removed and tested for Lercanidipine at intervals.
In the pharmacokinetic analysis of the composition 2 according to Example 2, the racemic mixture of Lercanidipine is measured. Table 4 shows the main pharmacokinetic parameters obtained from the test.

**TABLE 4: Pharmacokinetic analysis of composition 2 versus reference product (B)**

| | lnAUC0-t (ng h/ml) | lnAUC0-inf (ng h/ml) | Cmax (ng/ml) |
|---|---|---|---|
| Ratio of least square means (comp2/B)% | 84.0 | 87.0 | 125.0 |
| 90% C.I. (comp2B)% | 69.0 to 102.0 | 71.0 to 106.0 | 105.0 to 149.0 |
| Intra subject CV% | 27.3 | 27.8 | 24.1 |

Consequently, it has been found that composition 2 (20 mg tablet) has a relative bioavailability of 84% compared to the marketed Lercanidipine HCl 20 mg tablet.

Further, a single-dose study was conducted in 72 human volunteers in a randomised two-way crossover study, comparing the dosage form of composition of Example 3 with the dosage form of composition B. Plasma samples were removed and tested for Lercanidipine at intervals.

In the pharmacokinetic analysis of the composition 3 according to Example 3, R-Lercanidipine and S-Lercanidipine are measured separately (chiral method). Table 5 shows the main pharmacokinetic parameters obtained from the test.

**TABLE 5: Pharmacokinetic analysis of composition 3 versus reference product (B)**

| | lnAUC0-t (ng h/ml) | lnAUC0-inf (ng h/ml) | Cmax (ng/ml) |
|---|---|---|---|
| R-Lercanidipine | | | |
| Ratio of least square means (comp3/B)% | 143.0 | 140.6 | 187.1 |
| 90% C.I. (omp3/B)% | 129.6 to 157.7 | 128.0 to 154.6 | 168.2 to 208.1 |
| Intra subject CV% | 34.5 | 32.8 | 37.5 |

| S-Lercanidipine | | | |
|---|---|---|---|
| Ratio of least square means (comp3/B)% | 144.4 | 142.5 | 181.1 |
| 90% C.I. (comp3/B)% | 131.8 to 158.2 | 130.4 to 155.7 | 164.0 to 200.0 |
| Intra subject CV% | 31.9 | 30.7 | 34.8 |

Consequently, it has been found that composition 3 (20 mg tablet) has a relative bioavailability of approximately 144% compared to the marketed Lercanidipine HCl 20 mg tablet (Fig 3 and 4).

The in vivo results indicate that concentration level of the active substance in plasma for composition 1 is approximately 50% of the level of the reference product. Composition 2 showed 68% increase in plasma concentration in comparison with composition 1. Surprisingly, composition 3 that is at approximately 144% of the level of the reference product has an increase of 71% and 188% in comparison with compositions 2 and 1, respectively.

The composition 3 of the present invention can therefore be considered, with respect the pharmacological performance, the best bioavailable solid formulations currently available.

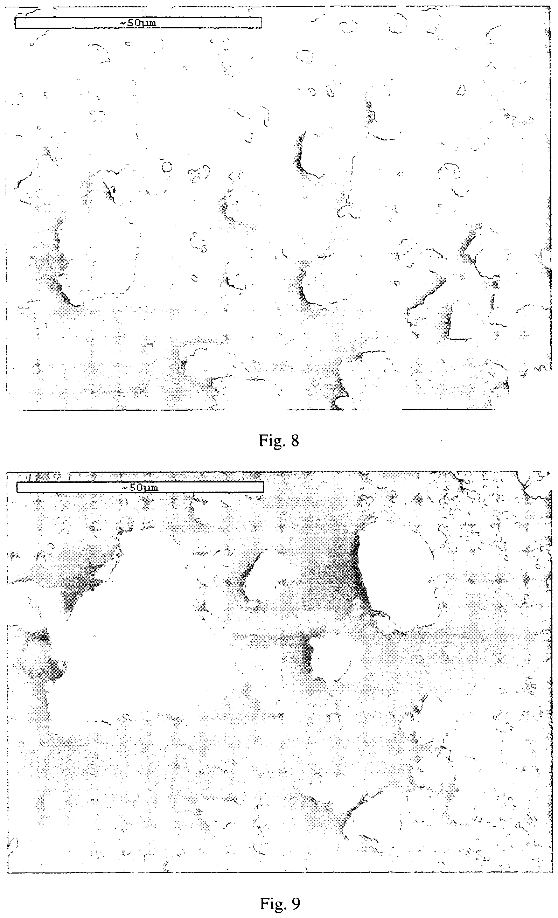

The increased bioavailability of composition 3 can be attributed to the formation of a fine dispersion between colloidal silica dioxide and Lercanidipine HCl. The optimal dispersion was verified by scanning electron microscopy (SEM) analysis and as it can be seen from Fig. 8 and 9 no particles or agglomerates of the drug substance were observed.

The very large surface of Aerosil™, on which the active ingredient is absorbed, results to an increase of the specific surface area and contribute to the excess of bioavailability.

The presence of the binder facilitates the homogenous distribution of the active ingredient on the surface of the Aerosil™ particles.

Even though the same dose (20mg) was administered to the volunteers, composition 3 showed an enhanced bioavailability in comparison with the marketed reference product.

This fact gives the possibility to manufacture a pharmaceutical composition with smaller quantity of active ingredient than the reference product but with the same effect resulting in better patient compliance and less side effects.

## Claims

1. A process for the preparation of a solid dosage form for oral administration such as a tablet, capsule or sachet containing dihydropyridine calcium channel antagonist Lercanidipine or a pharmaceutical acceptable salt or polymorph thereof as an active ingredient and an amount, from 5% to 25% by weight, of colloidal silicon dioxide, which comprises:
- dissolving the total quantity of said active ingredient, a portion of the total quantity of said colloidal silicon dioxide to enhance bioavailability and a binder into water/EtOH solvent;
- adding to the formed solution the remaining portion of colloidal silicon dioxide and an optional excipient such as a diluent, a binder, a disintegrant, a glidant, a lubricant and wet granulating;
- dissolving a wetting agent into a small quantity of water/EtOH solvent and kneading with the first solution;
- drying the wetted mass;
- sieving the dried mass and adding to the sieved mixture the total quantities of at least one optional excipient such as a binder, a diluent, a disintegrant, a lubricant and/or a glidant and mixing until uniform, and
- formulating the resulting mixture in a solid dosage form either by compressing it into a desired tablet form or by filling capsules or sachets.

2. A pharmaceutical composition for oral administration obtainable by the process of claim 1.

3. The pharmaceutical composition according to claim 2, wherein the weight ratio of dihydropyridine calcium channel antagonist Lercanidipine or a pharmaceutical acceptable salt or polymorph thereof to colloidal silicon dioxide is from 10/1 to 1/60.

4. The pharmaceutical composition according to claim 2, wherein it comprises 0.5% to 30%, by weight of dihydropyridine calcium channel antagonist Lercanidipine or a pharmaceutical acceptable salt or polymorph thereof.

5. The pharmaceutical composition according to claim 4, wherein it comprises 3% to 25% by weight of dihydropyridine calcium channel antagonist Lercanidipine or a pharmaceutical acceptable salt or polymorph thereof

6. The pharmaceutical composition according to claim 4, wherein it comprises 5% to 10% by weight of dihydropyridine calcium channel antagonist Lercanidipine or a pharmaceutical acceptable salt or polymorph thereof

7. The pharmaceutical composition according to any of claims 2 to 6, wherein it comprises 7% to 20% by weight of colloidal silicon dioxide.

8. The pharmaceutical composition according to any of claims 2 to 7, wherein it further comprises a binder such as PVP.

9. The pharmaceutical composition according to any of claims 2 to 8, wherein it further comprises at least one optionally excipient selected from the group consisting of diluents, binders, disintegrants, lubricants, and glidants.

10. The pharmaceutical composition according to any of claims 2 to 9, wherein said composition is in a solid dosage form such as a tablet, capsule or sachet.

11. The pharmaceutical composition according to any of claims 2 to 10, wherein said composition is sustained release.

12. The pharmaceutical composition according to any of claims 2 to 10, wherein said composition is immediate release.

## Patentansprüche

1. Verfahren zur Herstellung von einer festen Darreichungsform zur oralen Verabreichung, wie Tablette, Kapsel oder Beutel, der Dihydropyridin-Calciumkanal-Antagonist Lercanidipin oder ein pharmazeutisch annehmbares Salz oder Polymorph davon als ein aktives Mittel und einer Menge von 5% bis 25% Gewichts kolloidalem Siliciumdioxid enthält, wobei unfasst:
- der Gesamtmenge des aktiven Wirkstoffs, und eines Teils der Gesamtmenge des kolloidalen Siliciumdioxids, um die Bioverfügbarkeit zu verbessern, und ein Bindemittel in Wasser / EtOH-Lösungsmittel auflösen;
- des verbleibenden Teils von kolloidalem Siliciumdioxid und eines optionalen Hilfsstoffs, wie ein Verdünnungsmittel, ein Bindemittel, ein Sprengmittel, ein Gleitmittel, ein Schmiermittel zu der gebildeten Lösung zugaben und feuchtgranulieren;
- eines Netzmittels in eine kleine Menge von Wasser / EtOH als Lösungsmittel auflösen und mit der ersten Lösung kneten;
- der benetzten Masse trocknen;
- der getrockneten Masse sieben und der Gesamtmengen mindestens eines optionalen Hilfsstoffs, wie eines Bindemittels, eines Verdünnungsmittels, eines Sprengmittels, eines Schmiermittels und / oder eines Gleitmittels zu dem gesiebten Gemisch zugaben und bis es gleichmäßig ist, mischen, und
- der resultierenden Mischung in einer festen Dosierungsform formulieren entweder durch in eine gewünschte Tablettenform komprimieren oder durch von Kapseln oder Sachets füllen.

2. Pharmazeutische Zusammensetzung zur oralen Verabreichung, die durch das Verfahren nach Anspruch 1 erhältlich ist.

3. Pharmazeutische Zusammensetzung nach Anspruch 2, wobei das Gewichtsverhältnis des Dihydropyridin-Calciumkanal-Antagonisten Lercanidipin oder eines pharmazeutisch annehmbares Salzes oder Polymorphs davon zu kolloidalem Siliciumdioxid von 10/1 bis 1/60 ist.

4. Pharmazeutische Zusammensetzung nach Anspruch 2, wobei sie 0,5% bis 30% Gewichts Dihydropyridin-Calciumkanal-Antagonist Lercanidipin oder ein pharmazeutisch annehmbares Salz oder Polymorph davon umfasst.

5. Pharmazeutische Zusammensetzung nach Anspruch 4, wobei sie 3% bis 25% Gewichts Dihydropyridin-Calciumkanal-Antagonist Lercanidipin oder ein pharmazeutisch annehmbares Salz oder Polymorph davon umfasst.

6. Pharmazeutische Zusammensetzung nach Anspruch 4, wobei sie 5% bis 10% Gewichts Dihydropyridin-Calciumkanal-Antagonist Lercanidipin oder ein pharmazeutisch annehmbares Salz oder Polymorph davon umfasst.

7. Pharmazeutische Zusammensetzung nach einem der Ansprüche 2 bis 6, wobei sie 7% bis 20% Gewichts kolloidales Siliciumdioxid enthält.

8. Pharmazeutische Zusammensetzung nach einem der Ansprüche 2 bis 7, wobei sie ferner ein Bindemittel, wie PVP, umfasst.

9. Pharmazeutische Zusammensetzung nach einem der Ansprüche 2 bis 8, wobei sie ferner mindestens einen optionalen Hilfsstoff umfasst, ausgewählt aus der Gruppe bestehend aus Verdünnungsmitteln, Bindemitteln, Sprengmitteln, Schmiermitteln und Gleitmitteln.

10. Pharmazeutische Zusammensetzung nach einem der Ansprüche 2 bis 9, wobei die genannte Zusammensetzung in einer festen Dosierungsform, wie einer Tablette, einer Kapsel oder einem Beutel ist.

11. Pharmazeutische Zusammensetzung nach einem der Ansprüche 2 bis 10, wobei die genannte Zusammensetzung mit verlängerter Freisetzung ist.

12. Pharmazeutische Zusammensetzung nach einem der Ansprüche 2 bis 10, wobei die genannte Zusammensetzung mit sofortiger Freisetzung ist.

## Revendications

1. Un procédé pour la préparation d'une forme posologique solide pour administration par voie orale telle qu'un comprimé, une gélule ou un sachet contenant l'antagoniste de canaux de calcium de type dihydropyridine: la Lercanidipine ou un sel pharmaceutiquement acceptable ou polymorphe de celle-ci comme principe actif et une quantité, de 5% à 25% en masse, de dioxyde de silicium colloïdal comprenant :
- dissoudre la quantité totale du dit principe actif, une part de la quantité totale du dit dioxyde de silicium colloïdal pour accroître la biodisponibilité et un liant dans eau/EtOH solvant;
- ajouter à la solution formée la portion restante du dioxyde de silicium colloïdal et un excipient optionnel tel qu'un diluant, un liant, un désintégrant, un agent glissant, un lubrifiant et un agent de granulation par voie humide;
- dissoudre un agent mouillant dans une petite quantité d'eau/EtOH solvant et malaxer avec la première solution;
- sécher la masse humide;
- tamiser la masse séchée et ajouter au mélange tamisé les quantités totales d'au moins un excipient optionnel tel qu'un liant, un diluant, un désintégrant, un lubrifiant et/ou un agent glissant et mélanger uniformément; et
- formuler le mélange résultant sous une forme posologique solide soit en le compressant sous forme de comprimé soit en le remplissant dans des gélules ou sachets.

2. Composition pharmaceutique pour administration par voie orale pouvant être obtenue par le procédé selon la revendication 1.

3. La composition pharmaceutique selon la revendication 2, où le rapport pondéral de l'antagoniste de canaux de calcium de type dihydropyridine: la Lercanidipine ou un sel pharmaceutiquement acceptable ou polymorphe de celle-ci au dioxyde de silicium colloïdal est de 10/1 à 1/60.

4. La composition pharmaceutique selon la revendication 2, dans laquelle est compris de 0.5% à 30% en masse d'antagoniste de canaux de calcium de type dihydropyridine:
la Lercanidipine ou un sel pharmaceutiquement acceptable ou un polymorphe de celle-ci.

5. La composition pharmaceutique selon la revendication 4, dans laquelle est compris de 3% à 25% en masse d'antagoniste de canaux de calcium de type dihydropyridine:
la Lercanidipine ou un sel pharmaceutiquement acceptable ou un polymorphe de celle-ci.

6. La composition pharmaceutique selon la revendication 4, dans laquelle est compris de 5% à 10% en masse d'antagoniste de canaux de calcium de type dihydropyridine:
la Lercanidipine ou un sel pharmaceutiquement acceptable ou un polymorphe de celle-ci.

7. La composition pharmaceutique selon l'une quelconque des revendications de 2 à 6, dans laquelle est compris de 7% à 20% en masse de dioxyde de silicium colloïdal.

8. La composition pharmaceutique selon l'une quelconque des revendications de 2 à 7, dans laquelle est compris en outre un liant tel que la PVP.

9. La composition pharmaceutique selon l'une quelconque des revendications de 2 à 8, dans laquelle est compris en outre au moins un excipient optionnel sélectionné parmi le groupe composé de diluants, de liants, de désintégrants, de lubrifiants et d'agents glissant.

10. La composition pharmaceutique selon l'une quelconque des revendications de 2 à 9, où la dite composition est sous forme posologique solide tel qu'un comprimé, une gélule ou un sachet.

11. La composition pharmaceutique selon l'une quelconque des revendications de 2 à 10, où la dite composition est à libération prolongée.

12. La composition pharmaceutique selon l'une quelconque des revendications de 2 à 10, où la dite composition est à libération immédiate.
